# EUROPEAN PATENT APPLICATION

(11) **EP 4 353 243 A1**
(43) Date of publication of application: **17.04.2024**
(21) Application number: 22819909.7
(22) Date of filing: 28.03.2022
(51) Int. Cl.: A61K 35/28, A61K 35/545, A61P 1/16, A61P 29/00, C12N 5/0775, C12N 5/10

(54) **METHOD FOR PRODUCING MESENCHYMAL STEM CELLS**

(30) Priority: 10.06.2021 JP 2021097619
(71) Applicant: AJINOMOTO CO., INC., Chuo-ku Tokyo 104-8315 (JP)
(72) Inventor: TAKAHASHI, Kazuma, Kawasaki-shi, Kanagawa 210-8681 (JP); BABA, Shizuka, Kawasaki-shi, Kanagawa 210-8681 (JP); KONISHI, Atsushi, Kawasaki-shi, Kanagawa 210-8681 (JP)
(74) Representative: Hoffmann Eitle
(86) International application number: PCT/JP2022/015032
(87) International publication number: WO 2022/259721

(57) **Abstract**

The present invention provides a medium composition for efficient preparation of mesenchymal stem cells, a method for producing mesenchymal stem cells, a medicament for the prophylaxis or treatment of inflammatory diseases or fibrous diseases, and the like. Specifically, a method for producing a medicament for the prophylaxis or treatment of inflammatory diseases or fibrous diseases, comprising a step of inducing mesenchymal stem cells by culturing neural crest cells in a medium composition for inducing mesenchymal stem cells from neural crest cells, comprising a basal medium and corticosteroid, the composition having a converted concentration of the corticosteroid in the medium composition of not less than 1.25 µM is provided.

## Description

### [Technical Field]

The present invention relates to production methods of mesenchymal stem cells and the like. More particularly, the present invention relates to production methods of mesenchymal stem cells, using medium composition containing basal medium and corticosteroid and the like.

### [Background Art]

Mesenchymal stem cell (MSC) is one type of somatic stem cells present in the bone marrow and the like of a living body, and is defined as an adherent cell having an ability to differentiate into bone, cartilage or adipocyte. Mesenchymal stem cell is considered to have an extremely low risk of canceration, and considered to be highly promising as a cellular material to be used for regenerative medicine. In addition, it is known that mesenchymal stem cells accumulate at tissue injury sites, have a function to control immune responses and anti-inflammatory actions by releasing various humoral factors and exosomes, and play an important role in tissue repair and maintaining homeostasis. Therefore, mesenchymal stem cells themselves are promising as cell preparations for treating immune diseases, inflammatory diseases, and the like.

However, since mesenchymal stem cells have different differentiation potency and proliferation/functional properties depending on the tissue of origin and the like, it is necessary to efficiently produce mesenchymal stem cells that exhibit useful properties according to the purposes.

Neural crest cell is a cell unique to vertebrates, which occurs transiently between the neural tube and the presumptive epidermal ectoderm during an early stage of development, migrates throughout the embryo, and differentiates into a wide variety of cells such as cells of the peripheral nervous system, cells of the head tissue, pigment cells, and the like. While it has been known that neural crest cells can be produced by the methods disclosed in Patent Literature 1 and Non Patent Literature 1, and that differentiation of mesenchymal stem cells can be induced from them (Non Patent Literature 2), a method for more efficiently producing mesenchymal stem cells has been studied.

### [Citation List]

### [Patent Literature]

[PTL 1]
WO 2020/230832

### [Non Patent Literature]

[NPL 1] Fukuta M. et al., PLoS One. 2014 Dec 2; 9(12):e 112291.
[NPL 2] Zhao C. and Ikeya M., Stem Cells Int. 2018 Jul 31; Article ID 9601623

### [Summary of Invention]

### [Technical Problem]

The problem of the present invention is to provide a medium composition, a production method of mesenchymal stem cells, and the like for efficiently producing mesenchymal stem cells.

### [Solution to Problem]

The present inventors have conducted intensive studied the above-mentioned problems and found that mesenchymal stem cells can be efficiently induced from neural crest cells by using a medium composition containing corticosteroid, and have also found a suitable concentration of corticosteroid in the medium composition. Furthermore, they have found that mesenchymal stem cells obtained by culturing neural crest cells in such a medium composition highly express TSG-6 and are useful as a medicament for the prophylaxis or treatment of inflammatory diseases. In addition, they have found that the secretome of mesenchymal stem cells obtained by culturing neural crest cells in such a medium composition reduces the expression of COL1A1 and ACTA2, which are marker genes for fibrosis, and therefore useful as a medicament for the prophylaxis or treatment of fibrous diseases, which resulted in the completion of the present invention.

Accordingly, the present invention provides the following.
[1] A method for producing a medicament for the prophylaxis or treatment of inflammatory diseases or fibrous diseases, comprising a step of inducing mesenchymal stem cells by culturing neural crest cells in a medium composition for inducing mesenchymal stem cells from neural crest cells, comprising a basal medium and corticosteroid, the composition having a converted concentration of the corticosteroid in the medium composition of not less than 1.25 µM.
[2] The method of [1], wherein the medicament for the prophylaxis or treatment of inflammatory diseases or fibrous diseases comprises a mesenchymal stem cell characterized by high expression of TSG-6 as an active ingredient.
[3] The method of [1], which is a method for producing a medicament for the prophylaxis or treatment of inflammatory diseases, further comprising collecting TSG-6 protein from the induced mesenchymal stem cells and/or culture supernatant, wherein the medicament for the prophylaxis or treatment of inflammatory diseases comprises TSG-6 protein as an active ingredient.
[4] The method of [1], which is a method for producing a medicament for the prophylaxis or treatment of fibrous diseases, further comprising collecting secretome from the induced mesenchymal stem cells and/or culture supernatant, wherein the medicament for the prophylaxis or treatment of fibrosis comprises the secretome as an active ingredient.
[5] The production method of any of [1] to [4], wherein the corticosteroid is glucocorticoid or a derivative thereof.
[6] The production method of [5], wherein the glucocorticoid or a derivative thereof is at least one member selected from the group consisting of cortisone acetate, hydrocortisone, fludrocortisone acetate, predonisolone, triamcinolone, methylprednisolone, dexamethasone, betamethasone, and beclometasone dipropionate.
[7] The production method of [5] or [6], wherein the glucocorticoid or a derivative thereof is dexamethasone.
[8] The production method of any of [1] to [7], wherein the converted concentration of the corticosteroid in the medium composition is not more than 300 µM.
[9] The production method of [7], wherein the concentration of dexamethasone in the medium composition is not more than 10 µM.
[10] The production method of any of [1] to [9], wherein the basal medium is a serum-free medium.
[11] The production method of any of [1] to [10], wherein the neural crest cell is derived from a pluripotent stem cell.
[12] The production method of [11], wherein the pluripotent stem cell is an induced pluripotent stem cell (iPS cell).
[13] The production method of any of [1] to [12], wherein the following steps are performed before the aforementioned step:
   (step A) a step of obtaining a cell population including neural crest cells, and
   (step B) a step of expansion culture of the cell population obtained in step A, using an extracellular matrix as a scaffold.
[14] The production method of [13], wherein the extracellular matrix is laminin or fibronectin.
[15] The production method of [14], wherein the laminin is a full-length laminin, laminin having an α2 chain or laminin 211.
[16] The production method of any of [13] to [15], wherein the cell population including neural crest cells obtained in step A is subjected to step B without being subjected to a neural crest cell sorting treatment.
[17] The production method of any of [1] to [3] and [5] to [16], wherein the inflammatory disease is cirrhosis.
[18] The production method of any of [2], [3] and [5] to [17], wherein the high expression of TSG-6 is a high expression in response to IFNγ stimulation.
[19] A medicament for the prophylaxis or treatment of inflammatory diseases produced by the production method of any of [1] to [3] and [5] to [18], or a medicament for the prophylaxis or treatment of fibrous diseases produced by the production method of any of [1], [2], [4] to [18].

### [Advantageous Effects of Invention]

According to the present invention, a medium composition for efficiently inducing mesenchymal stem cells from neural crest cells can be provided. According to the present invention, moreover, an efficient production method of mesenchymal stem cells, a medicament for the prophylaxis or treatment of inflammatory diseases or fibrous diseases, containing the mesenchymal stem cells and the like as active ingredients, a prophylactic or therapeutic method for the diseases, a production method of a medicament for the treatment, and the like can be provided.

### [Brief Description of Drawings]

[Fig. 1]

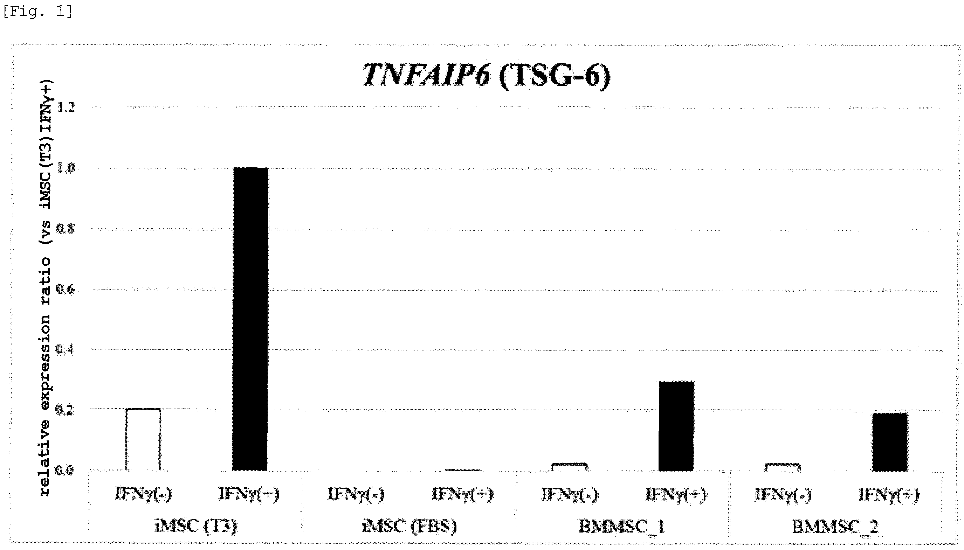

   Fig. 1 is a graph showing the gene expression of TNFAIP6 (TSG-6) after stimulation at 50 ng/mL IFNγ for 48 hr in various mesenchymal stem cells.
[Fig. 2]

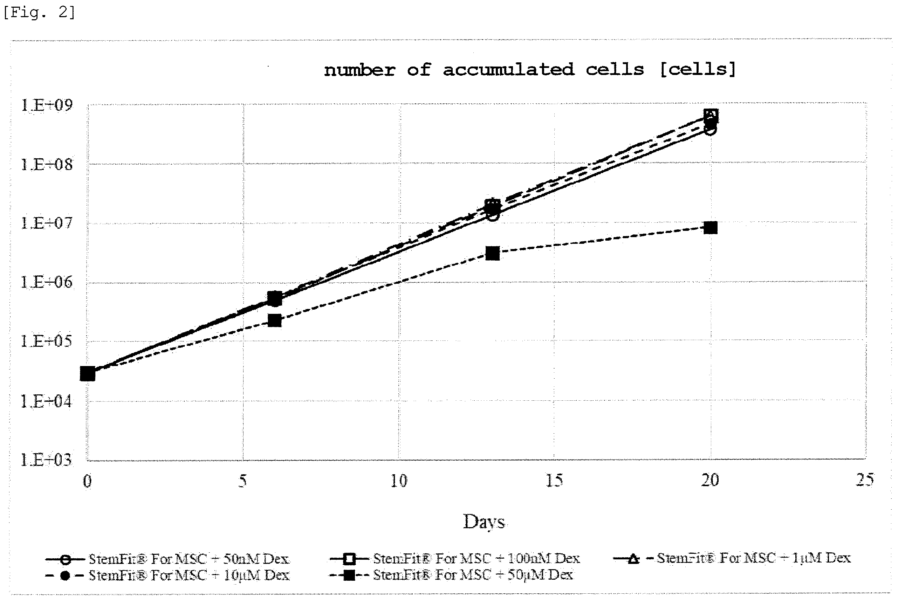

   Fig. 2 is a graph showing the cell proliferation curve during differentiation induction.
[Fig. 3]

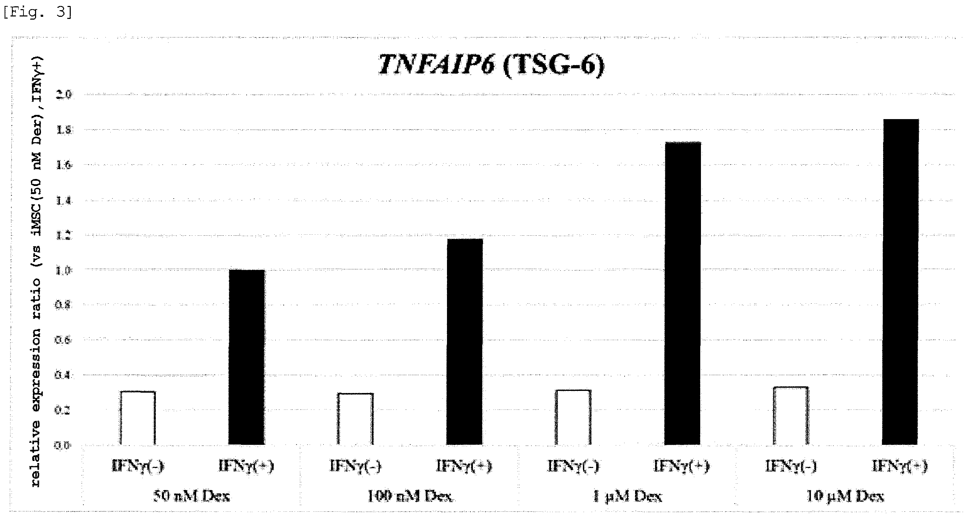

   Fig. 3 is a graph showing the gene expression of TNFAIP6 (TSG-6) after stimulation at 50 ng/mL IFNγ for 48 hr.
[Fig. 4]

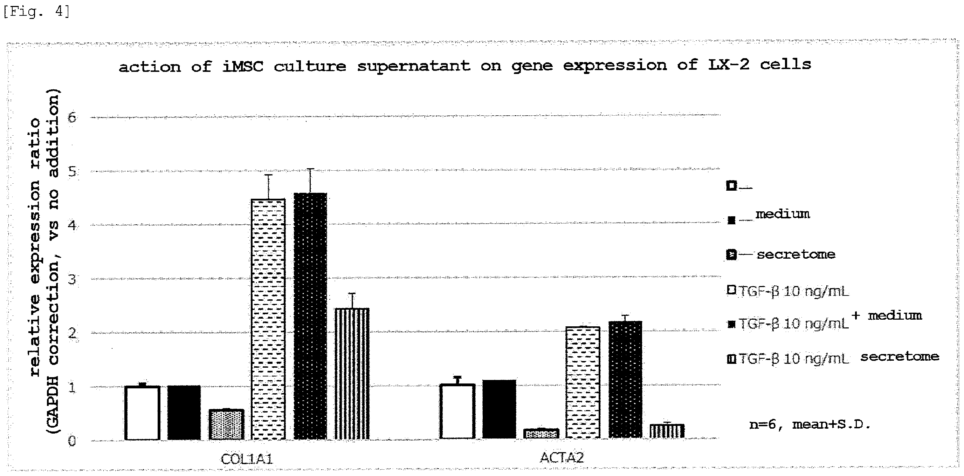

   Fig. 4 is a graph showing that the secretome isolated from the mesenchymal stem cells of the present invention markedly reduced the expression of COL1A1 and ACTA2, which are fibrosis marker genes, in the LX-2 cell line.

### [Description of Embodiments]

The present invention is explained in detail in the following.

### 1. Medium composition

The present invention provides a medium composition for inducing mesenchymal stem cells from neural crest cells, containing a basal medium and corticosteroid, the composition having a converted concentration of the corticosteroid in the medium composition of not less than 1.25 µM (hereinafter also to be referred to as the medium composition of the present invention).

As the basal medium contained in the medium composition of the present invention, a basal medium known per se can be used, and the medium is not particularly limited as long as it does not inhibit induction of neural crest cell into mesenchymal stem cells. The basal medium includes, for example, IMDM medium, Medium199 medium, Eagle's Minimum Essential medium (EMEM) medium, αMEM medium, Dulbecco's modified Eagle's medium (DMEM) medium, Ham's F12 medium, RPMI 1640 medium, Fischer's medium, StemPro34 (Invitrogen), RPMI-base medium, StemFit (registered trademark), MCDB201 medium and mixed medium of these, and the like. In this step, StemFit (registered trademark) medium is preferably used. The medium may contain a serum, or may be serum-free. Where necessary, the medium may contain one or more serum replacements such as albumin, transferrin, Knockout Serum Replacement (KSR) (serum replacement for FBS during ES cell culture), N2 supplement (Invitrogen), B27 supplement (Invitrogen), fatty acid, insulin, collagen precursor, trace element, 2-mercaptoethanol (2ME), thiolglycerol, and the like, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts, and the like.

The aforementioned medium contains corticosteroid. Examples of the corticosteroid include glucocorticoid, a derivative thereof, and the like and examples of the glucocorticoid and a derivative thereof include cortisone, cortisone acetate, hydrocortisone, fludrocortisone acetate, paramethasone, predonisolone, triamcinolone, methylprednisolone, dexamethasone, betamethasone, and beclometasone dipropionate. Among these, predonisolone, dexamethasone, or betamethasone is preferred, and dexamethasone is particularly preferred. One or more kinds of corticosteroids may be added to the medium, and one kind is preferably added.

Dexamethasone (CAS No.: 50-02-2, CA index name: Pregna-1,4-diene-3,20-dione,9-fluoro-11,17,21-trihydroxy-16-methyl-,(11β,16α)-) is a known synthetic corticosteroid having a structure represented by the following formula:

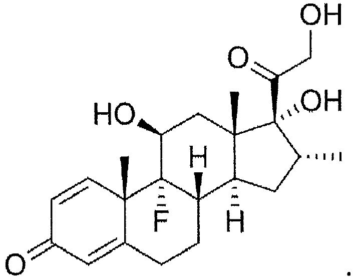

Since dexamethasone is commercially available, such commercially available product can also be used in the present invention. Since differentiation from neural crest cells into mesenchymal stem cells is induced by using dexamethasone, mesenchymal stem cells can be produced with high efficiency by culturing neural crest cells in the medium composition of the present invention.

The concentration of corticosteroid contained in the medium composition is not particularly limited as long as it is a concentration that can promote differentiation from neural crest cells into mesenchymal stem cells. For example, when only one type of dexamethasone is used, it is not less than 50 nM (or more than 50 nM), not less than 55 nM (or more than 55 nM), not less than 60 nM (or more than 60 nM), not less than 65 nM (or more than 65 nM), not less than 70 nM (or more than 70 nM), not less than 75 nM (or more than 75 nM), not less than 80 nM (or more than 80 nM), not less than 85 nM (or more than 85 nM), not less than 90 nM (or more than 90 nM), not less than 95 nM (or more than 95 nM), not less than 100 nM (or more than 100 nM), not less than 150 nM (or more than 150 nM), not less than 200 nM (or more than 200 nM), not less than 250 nM (or more than 250 nM), not less than 300 nM (or more than 300 nM), not less than 350 nM (or more than 350 nM), not less than 400 nM (or more than 400 nM), not less than 450 nM (or more than 450 nM), not less than 500 nM (or more than 500 nM), not less than 550 nM (or more than 550 nM), not less than 600 nM (or more than 600 nM), not less than 650 nM (or more than 650 nM), not less than 700 nM (or more than 700 nM), not less than 750 nM (or more than 750 nM), not less than 800 nM (or more than 800 nM), not less than 850 nM (or more than 850 nM), not less than 900 nM (or more than 900 nM), not less than 950 nM (or more than 950 nM), and not more than 10 µM (or less than 10 µM), not more than 9 µM (or less than 9 µM), not more than 8 µM (or less than 8 µM), not more than 7 µM (or less than 7 µM), not more than 6 µM (or less than 6 µM), not more than 5 µM (or less than 5 µM), not more than 4 µM (or less than 4 µM), not more than 3 µM (or less than 3 µM), not more than 2 µM (or less than 2 µM), not more than 1 µM (or less than 1 µM). When corticosteroid other than dexamethasone is used, it is desirably used at a concentration indicating a titer as a glucocorticoid equivalent to the aforementioned concentration of dexamethasone. When the concentration of corticosteroid in the medium is too low, it may not be sufficient to maintain the culture or promote the induction of mesenchymal stem cells, whereas when the concentration of corticosteroid is too high, it inhibits cell proliferation and mesenchymal stem cells cannot be obtained efficiently. For example, when differentiation into human mesenchymal stem cells is induced, the concentration of corticosteroid contained in the medium composition when only one type of dexamethasone is used is, for example, 50 nM to 10 µM (or not less than 50 nM and less than 10 µM, more than 50 nM and not more than 10 µM, or more than 50 nM and less than 10 µM), preferably 60 nM to 5 µM (or not less than 60 nM and less than 5 µM, more than 60 nM and not more than 5 µM, or more than 60 nM and less than 5 µM), more preferably 70 nM to 2 µM (or not less than 70 nM and less than 2 µM, more than 70 nM and not more than 2 µM, or more than 70 nM and less than 2 µM), further more preferably 100 nM to 1 µM (or not less than 100 nM and less than 1 µM, more than 100 nM and not more than 1 µM, or more than 100 nM and less than 1 µM).

The titer of corticosteroid as glucocorticoid when hydrocortisone is set to 1 is: cortisone 0.8, prednisolone 4, methylprednisolone 5, triamcinolone 5, paramethasone 10, dexamethasone 25 to 30, and betamethasone 25 to 30. When the titer of each corticosteroid as glucocorticoid may differ from the above-mentioned value depending on the manufacturer or storage conditions, it is desirable to confirm the titer by testing beforehand and to use a product of good quality under good storage conditions that has the above-mentioned value.

In addition, when only one type of betamethasone is used, the concentration of betamethasone contained in the medium composition is not particularly limited as long as it is a concentration that can promote differentiation from neural crest cells into mesenchymal stem cells, and is, for example, not less than 50 nM (or more than 50 nM), not less than 55 nM (or more than 55 nM), not less than 60 nM (or more than 60 nM), not less than 65 nM (or more than 65 nM), not less than 70 nM (or more than 70 nM), not less than 75 nM (or more than 75 nM), not less than 80 nM (or more than 80 nM), not less than 85 nM (or more than 85 nM), not less than 90 nM (or more than 90 nM), not less than 95 nM (or more than 95 nM), not less than 100 nM (or more than 100 nM), not less than 150 nM (or more than 150 nM), not less than 200 nM (or more than 200 nM), not less than 250 nM (or more than 250 nM), not less than 300 nM (or more than 300 nM), not less than 350 nM (or more than 350 nM), not less than 400 nM (or more than 400 nM), not less than 450 nM (or more than 450 nM), not less than 500 nM (or more than 500 nM), not less than 550 nM (or more than 550 nM), not less than 600 nM (or more than 600 nM), not less than 650 nM (or more than 650 nM), not less than 700 nM (or more than 700 nM), not less than 750 nM (or more than 750 nM), not less than 800 nM (or more than 800 nM), not less than 850 nM (or more than 850 nM), not less than 900 nM (or more than 900 nM), not less than 950 nM (or more than 950 nM), and not more than 10 µM (or less than 10 µM), not more than 9 µM (or less than 9 µM), not more than 8 µM (or less than 8 µM), not more than 7 µM (or less than 7 µM), not more than 6 µM (or less than 6 µM), not more than 5 µM (or less than 5 µM), not more than 4 µM (or less than 4 µM), not more than 3 µM (or less than 3 µM), not more than 2 µM (or less than 2 µM), not more than 1 µM (or less than 1 µM). When the concentration of betamethasone in the medium is too low, it may not be sufficient to maintain the culture or promote the induction of mesenchymal stem cells, whereas when the concentration of betamethasone is too high, it inhibits cell proliferation and mesenchymal stem cells cannot be obtained efficiently. For example, when differentiation into human mesenchymal stem cells is induced, the concentration of betamethasone contained in the medium composition is, for example, 50 nM to 10 µM (or not less than 50 nM and less than 10 µM, more than 50 nM and not more than 10 µM, or more than 50 nM and less than 10 µM), preferably 60 nM to 5 µM (or not less than 60 nM and less than 5 µM, more than 60 nM and not more than 5 µM, or more than 60 nM and less than 5 µM), more preferably 70 nM to 2 µM (or not less than 70 nM and less than 2 µM, more than 70 nM and not more than 2 µM, or more than 70 nM and less than 2 µM), further more preferably 100 nM to 1 µM (or not less than 100 nM and less than 1 µM, more than 100 nM and not more than 1 µM, or more than 100 nM and less than 1 µM).

In addition, when only one type of predonisolone is used, the concentration of predonisolone contained in the medium composition is not particularly limited as long as it is a concentration that can promote differentiation from neural crest cells into mesenchymal stem cells, and is, for example, not less than 300 nM (or more than 300 nM), not less than 330 nM (or more than 330 nM), not less than 360 nM (or more than 360 nM), not less than 390 nM (or more than 390 nM), not less than 420 nM (or more than 420 nM), not less than 450 nM (or more than 450 nM), not less than 480 nM (or more than 480 nM), not less than 510 nM (or more than 510 nM), not less than 540 nM (or more than 540 nM), not less than 570 nM (or more than 570 nM), not less than 600 nM (or more than 600 nM), not less than 900 nM (or more than 900 nM), not less than 1.2 µM (or more than 1.2 µM), not less than 1.5 µM (or more than 1.5 µM), not less than 1.8 µM (or more than 1.8 µM), not less than 2.1 µM (or more than 2.1 µM), not less than 2.4 µM (or more than 2.4 µM), not less than 2.7 µM (or more than 2.7 µM), not less than 3 µM (or more than 3 µM), not less than 3.3 µM (or more than 3.3 µM), not less than 3.6 µM (or more than 3.6 µM), not less than 3.9 µM (or more than 3.9 µM), not less than 4.2 µM (or more than 4.2 µM), not less than 4.5 µM (or more than 4.5 µM), not less than 4.8 µM (or more than 4.8 µM), not less than 5.1 µM (or more than 5.1 µM), not less than 5.4 µM (or more than 5.4 µM), not less than 5.7 µM (or more than 5.7 µM), and not more than 70 µM (or less than 70 µM), not more than 63 µM (or less than 63 µM), not more than 56 µM (or less than 56 µM), not more than 49 µM (or less than 49 µM), not more than 42 µM (or less than 42 µM), not more than 35 µM (or less than 35 µM), not more than 28 µM (or less than 28 µM), not more than 21 µM (or less than 21 µM), not more than 14 µM (or less than 14 µM), not more than 7 µM (or less than 7 µM). When the concentration of prednisolone in the medium is too low, it may not be sufficient to maintain the culture or promote the induction of mesenchymal stem cells, whereas when the concentration of prednisolone is too high, it inhibits cell proliferation and mesenchymal stem cells cannot be obtained efficiently. For example, when differentiation into human mesenchymal stem cells is induced, the concentration of prednisolone contained in the medium composition is, for example, 300 nM to 70 µM (or not less than 300 nM and less than 70 µM, more than 300 nM and not more than 70 µM, or more than 300 nM and less than 70 µM), preferably 360 nM to 35 µM (or not less than 360 nM and less than 35 µM, more than 360 nM and not more than 35 µM, or more than 360 nM and less than 35 µM), more preferably 420 nM to 14 µM (or not less than 420 nM and less than 14 µM, more than 420 nM and not more than 14 µM, or more than 420 nM and less than 14 µM), further more preferably 600 nM to 7 µM (or not less than 600 nM and less than 7 µM, more than 600 nM and not more than 7 µM, or more than 600 nM and less than 7 µM).

In one embodiment, the concentration of corticosteroid contained in the medium composition when only one type of each is used is not less than 50 nM and not more than 10 µM (or not less than 50 nM and less than 10 µM, more than 50 nM and not more than 10 µM, or more than 50 nM and less than 10 µM) for dexamethasone or betamethasone, not less than 100 nM and not more than 30 µM (or not less than 100 nM and less than 30 µM, more than 100 nM and not more than 30 µM, or more than 100 nM and less than 30 µM) for paramethasone, not less than 250 nM and not more than 60 µM (or not less than 250 nM and less than 60 µM, more than 250 nM and not more than 60 µM, or more than 250 nM and less than 60 µM) for triamcinolone or methylprednisolone, not less than 300 nM and not more than 70 µM (or not less than 300 nM and less than 70 µM, more than 300 nM and not more than 70 µM, or more than 300 nM and less than 70 µM) for predonisolone, not less than 1.50 µM and not more than 375 µM (or not less than 1.50 µM and less than 375 µM, more than 1.50 µM and not more than 375 µM, or more than 1.50 µM and less than 375 µM) for cortisone, and not less than 1.250 µM and not more than 300 µM (or not less than 1.250 µM and less than 300 µM, more than 1.250 µM and not more than 300 µM, or more than 1.250 µM and less than 300 µM) for hydrocortisone.

In one embodiment, the concentration of corticosteroid contained in the medium composition when only one type of each is used is not less than 50 nM and not more than 375 µM (or not less than 50 nM and less than 375 µM, more than 50 nM and not more than 375 µM, or more than 50 nM and less than 375 µM).

In one embodiment, when using one or more types of corticosteroids, the concentrations thereof are converted by multiplying the titer as glucocorticoid, and the total concentration thereof is defined as the converted concentration of the one or more types of corticosteroids. The converted concentration is, for example, not less than 1.25 µM (or more than 1.25 µM), not less than 1.375 µM (or more than 1.375 µM), not less than 1.5 µM (or more than 1.5 µM), not less than 1.625 µM (or more than 1.625 µM), not less than 1.75 µM (or more than 1.75 µM), not less than 1.875 µM (or more than 1.875 µM), not less than 2 µM (or more than 2 µM), not less than 2.125 µM (or more than 2.125 µM), not less than 2.25 µM (or more than 2.25 µM), not less than 2.375 µM (or more than 2.375 µM), not less than 2.5 µM (or more than 2.5 µM), not less than 3.75 µM (or more than 3.75 µM), not less than 5 µM (or more than 5 µM), not less than 6.25 µM (or more than 6.25 µM), not less than 7.5 µM (or more than 7.5 µM), not less than 8.75 µM (or more than 8.75 µM), not less than 10 µM (or more than 10 µM), not less than 11.25 µM (or more than 11.25 µM), not less than 12.5 µM (or more than 12.5 µM), not less than 13.75 µM (or more than 13.75 µM), not less than 15 µM (or more than 15 µM), not less than 16.25 µM (or more than 16.25 µM), not less than 17.5 µM (or more than 17.5 µM), not less than 18.75 µM (or more than 18.75 µM), not less than 20 µM (or more than 20 µM), not less than 21.25 µM (or more than 21.25 µM), not less than 22.5 µM (or more than 22.5 µM), not less than 23.75 µM (or more than 23.75 µM), and not more than 300 µM (or less than 300 µM), not more than 270 µM (or less than 270 µM), not more than 240 µM (or less than 240 µM), not more than 210 µM (or less than 210 µM), not more than 180 µM (or less than 180 µM), not more than 150 µM (or less than 150 µM), not more than 120 µM (or less than 120 µM), not more than 90 µM (or less than 90 µM), not more than 60 µM (or less than 60 µM), not more than 30 µM (or less than 30 µM). For example, when one or more types of corticosteroids are used, the converted concentration of the corticosteroids is 1.25 µM to 300 µM (or not less than 1.25 µM and less than 300 µM, more than 1.25 µM and not more than 300 µM, or more than 1.25 µM and less than 300 µM), preferably 1.5 µM to 150 µM (or not less than 1.5 µM and less than 150 µM, more than 1.5 µM and not more than 150 µM, or more than 1.5 µM and less than 150 µM), more preferably 17.5 µM to 60 µM (or not less than 17.5 µM and less than 60 µM, more than 17.5 µM and not more than 60 µM, or not less than 17.5 µM and less than 60 µM), further more preferably 2.5 µM to 30 µM (or not less than 2.5 µM and less than 30 µM, more than 2.5 µM and not more than 30 µM, or more than 2.5 µM and less than 30 µM).

The medium composition of the present invention may contain a serum. Serum is not particularly limited as long as it is derived from an animal and does not inhibit the induction of mesenchymal stem cells. Preferred is a mammal-derived serum (e.g., fetal bovine serum, human serum etc.). The concentration of the serum may be any as long as it is within a concentration range known per se. Furthermore, when a mesenchymal stem cell after culture is used for medical purposes, a serum-free medium can also be preferably used, since a component derived from other animal may be a blood-mediated pathogen or a xenoantigen. When serum is not contained, a replacement additive of serum (e.g., Knockout Serum Replacement (KSR) (Invitrogen), Chemically-defined Lipid concentrated (Gibco) etc.) may also be used.

When mesenchymal stem cells induced by the medium composition of the present invention are used for medical purposes such as cell medicine and the like, the medium of the present invention more preferably free of a component derived from a non-human animal, in view of the possibility of causing infection with pathogenic bacteria or becoming a xenoantigen.

The "stem cell" means an immature cell having self-renewal capacity and differentiation/growth capacity. The stem cell includes subpopulation such as pluripotent stem cell, multipotent stem cell, unipotent stem cell and the like, according to the differentiation potency. The pluripotent stem cell means a cell capable of differentiating into any tissue or cell constituting living organisms. The multipotent stem cell means a cell capable of differentiating into plural, though not all, kinds of tissues and cells. The unipotent stem cell means a cell capable of differentiating into specific tissues and cells.

The mesenchymal stem cell of interest in the present invention is one kind of multipotent stem cell which can differentiate into adipocyte, osteocyte, chondrocyte, muscle cell, hepatocyte, nerve cell, and the like, and is known as a cell having a low possibility of forming a tumor when transplanted into a living body. The mesenchymal stem cell in the present invention can be preferably positive to one or more mesenchymal stem cell markers (e.g., CD90, CD44, CD73, CD105, etc), more preferably said marker-positive, with expression of the molecule showing no expression in mesenchymal stem cell being negative. Examples of the molecule showing no expression in mesenchymal stem cell include CD34, CD45, CD14, CD11b, CD79, CD19, HLA-DR and the like.

The medium composition of the present invention can be preferably used for the induction of mesenchymal stem cells derived from any animals. The mesenchymal stem cells that can be induced and culture using the medium composition of the present invention are, for example, mesenchymal stem cells derived from rodents such as mouse, rat, hamster, guinea pig and the like, Lagomorpha such as rabbit and the like, Ungulata such as swine, bovine, goat, horse, sheep and the like, Carnivora such as dog, cat and the like, primates such as human, monkey, Macaca mulatta, marmoset, orangutan, chimpanzee and the like. Preferred are mesenchymal stem cells derived from human.

The medium composition of the present invention may contain factors inducing differentiation of mesenchymal stem cells (e.g., glucocorticoid, factor called transforming growth factor-β family, for example, bone morphogenetic protein (desirably BMP-2 or BMP-4), basic fibroblast growth factor (bFGF), inhibin A or chondrogenic stimulating stimulation activity factor (CSA), collagen extracellular substrate such as Type I collagen (particularly in gel form), and the like, and vitamin A analogs such as retinoic acid and the like) preferably at a concentration that does not induce differentiation of mesenchymal stem cell (e.g., differentiation into cartilage). In one embodiment, the medium may not contain factors other than corticosteroid that induce differentiation of mesenchymal stem cells. In one embodiment, the medium may not contain factors other than dexamethasone that induce differentiation of mesenchymal stem cells.

The medium composition of the present invention may contain a scaffold component. Examples of the scaffold component include laminin [including laminin α5β1γ1 (hereinafter laminin 511), laminin α1β1γ1 (hereinafter laminin 111), etc., and laminin fragments (laminin 511E8, etc.)], entactin, fibronectin, gelatin, vitronectin, Synthemax (Corning Incorporated), extracellular matrix such as Matrigel and the like, and the like, with preference given to laminin 511. These scaffold components are generally added to a medium at 0.001 ug/ml to 1000 µg/ml, preferably 0.01 ug/ml to 100 µg/ml, more preferably 0.1 µg/ml to 10 µg/ml, more preferably 0.1 µg/ml to 1 ug/ml, most preferably 0.2 µg/ml.

The medium composition of the present invention is used to induce differentiation of neural crest cells into mesenchymal stem cells.

The "neural crest cell (also referred to as "NCC")" means a cell that de-epithelializes from the neural crest, which is a structure temporarily formed between the epidermal ectoderm and neural plate in an early stage of development of vertebrates, and is induced to various sites within the embryonic body after the transition from epithelium to mesenchyme. The term "neural crest cell" in the present specification includes not only cells collected from a living body, but also neural crest cells derived from pluripotent stem cells and passaged cells thereof. In the present invention, the origin of neural crest cells is not particularly limited and they may be from any vertebrate, with preference given to neural crest cells derived from mammals. Such mammals include, but are not limited to, mouse, rat, guinea pig, hamster, rabbit, cat, dog, sheep, swine, bovine, horse, goat, monkey, and human, preferably human.

Neural crest cells to be used may be derived from a living body, and can be produced from tissues derived from the neural crest in a living body (e.g., bone marrow, dorsal root ganglion, heart, cornea, iris, dental pulp, olfactory mucosa, etc.). In addition, neural crest cells derived from pluripotent stem cells can also be used preferably. As a method for obtaining neural crest cells from pluripotent stem cells, methods known per se can be used. One example is a method including culturing pluripotent stem cells in a culture medium containing a TGFβ inhibitor and a GSK-3β inhibitor, in order to induce differentiation. By using methods known per se, it is possible to easily obtain neural crest cells, more specifically, a cell population containing neural crest cells.

Whether or not the thus-obtained cell population contains neural crest cells can be determined by confirming the expression of one or more neural crest cell-specific marker genes such as TFAP2a, SOX9, SOX10, TWISTI, and PAX3 by a method known per se. In addition, proteins present on the cell surface of neural crest cells such as CD271 protein (also referred to as "p75(NTR)") can also be used as neural crest cell-specific markers. In the present invention, neural crest cells are preferably derived from pluripotent stem cells, and more preferably from iPS cells.

Pluripotent stem cells include any cells that are induced into intermediate mesoderm cells that also have proliferation potency. Examples of the pluripotent stem cell include, but are not limited to, embryonic stem (ES) cell, embryonic stem (ntES) cell derived from cloned embryo obtained by nucleus transplantation, spermatozoon stem cell (GS cell), embryonic germ cell (EG cell), induced pluripotent stem (iPS) cell, pluripotent cell derived from cultured fibroblast or bone marrow stem cell (Muse cell), and the like. Preferred pluripotent stem cell is iPS cell, more preferably human iPS cell, from the viewpoint that they can be obtained without destroying embryo, ovum, and the like during the production step.

Methods for producing iPS cells are known in the art, and the cells can be produced by introducing reprogramming factors into any somatic cells. Examples of the reprogramming factor here include genes or gene products such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3, Glis1 and the like. These reprogramming factors may be used alone or in combination. Examples of the combination of reprogramming factors include the combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat. Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat. Cell Biol. 11:197-203, R.L. Judson et al., (2009), Nat. Biotechnol., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 47 4:225-9..

Somatic cells include, without limitation, all of fetal somatic cell, newborn baby somatic cell, and mature healthy or diseased somatic cell, and may also include any of primary cultured cell, passaged cell, and established cell line. Specifically, somatic cell includes, for example, (1) tissue stem cells (somatic stem cells) such as neural stem cell, hematopoietic stem cell, mesenchymal stem cell, pulp stem cell, and the like, (2) tissue progenitor cell, (3) differentiated cells such as blood cells (peripheral blood cell, cord blood cell, etc.), lymphocyte, epithelial cell, endothelial cell, muscle cell, fibroblast (skin cell, etc.), hair cell, hepatocyte, gastric mucosa cell, enterocyte, splenocyte, pancreatic cell (pancreatic exocrine cell, etc.), brain cell, lung cell, kidney cell, adipocyte, and the like, and the like.

The mammal from which the somatic cells are collected is not particularly limited, and is preferably a human.

The present invention can provide a differentiation promoter of mesenchymal stem cells, including corticosteroid. By culturing neural crest cells in a medium containing the differentiation promoter, the efficiency of induction into mesenchymal stem cells can be increased.

The differentiation promoter of the present invention may consist only of corticosteroid, but may also be provided as a composition further containing a physiologically acceptable carrier (e.g., physiological isotonic solution (physiological saline, the aforementioned basal medium, isotonic solution containing glucose or other auxiliary agent (e.g., D-sorbitol, D-mannitol, sodium chloride, and the like), etc.), excipient, antiseptic, stabilizer (e.g., human serum albumin, polyethylene glycol and the like), binder, solubilizing agent, non-ionic surfactant, and buffering agent (e.g., phosphate buffer, sodium acetate buffer), preservative, antioxidant, the aforementioned additive, and the like).

The content of corticosteroid contained in the differentiation promoter of the present invention is preferably constituted such that, when the differentiation promoter of the present invention is used by addition to a medium, the concentration of corticosteroid in the medium is sufficient to promote induction into mesenchymal stem cells.

The differentiation promoter of the present invention is used in the form of an isotonic aqueous solution or powder, which is added to a culture medium.

As described above, the medium composition of the present invention can be produced by adding corticosteroid to a basal medium, but it can also be used in the form of a kit containing corticosteroid and a basal medium. That is, the corticosteroid and the basal medium are separately combined and supplied in the form of a kit, and the user adds the corticosteroid to the basal medium at the time of use, thereby preparing and using the medium composition of the present invention.

In the kit, components constituting the basal medium and other components may be provided separately. Both components may be the same or different and may be liquids or powders, and each component may be provided singly or separately, or several components may be provided in a mixed state. In addition, the kit does not necessarily contain all the components of the medium composition of the present invention, and components that are extremely easily available, such as water, may be omitted. When the component is a powder, it can be dissolved in a buffer or the like and then used when desired.

### 2. Production method of mesenchymal stem cell

The present invention provides a production method of mesenchymal stem cells, including a step of culturing neural crest cells in the medium composition of the present invention to induce mesenchymal stem cells (step 1) (hereinafter to be also referred to as the production method of the present invention). In the production method of the present invention, differentiation into mesenchymal stem cells is induced by culturing neural crest cells in a medium composition containing a basal medium and corticosteroid. According to the production method of the present invention, mesenchymal stem cells can be produced efficiently. It is possible to efficiently differentiate neural crest cells into cells that retain the ability as mesenchymal stem cells, by culturing the neural crest cells in a medium composition containing corticosteroid.

The culture in step 1 may be suspension culture or adhesion culture or a combination of these. The "suspension culture" in the present invention refers to culturing while maintaining a state in which cells (or cell aggregates) are suspended in a culture medium solution. The "adhesion culture" refers to culturing under conditions that allow cells (or cell aggregates) to adhere to culture vessel material and the like. In this case, adhesion of cells refers to the formation of a strong cell-substratum junction between cells or cell aggregates and culture vessel material. More specifically, suspension culture refers to culture under conditions that do not allow strong cell-substratum junction to be formed between cells or cell aggregates and culture vessel material, etc., and adhesion culture refers to culture under conditions that allow strong cell-substratum junction to be formed between cells or cell aggregates and culture vessel material, etc.

The culture vessel used when performing suspension culture is not particularly limited as long as it is capable of suspension culture, and can be appropriately determined by those skilled in the art. Examples of such culture vessel include flask, tissue culture flask, culture dish, petri dish, tissue culture dish, multi-dish, microplate, microwell plate, micropore, multiplate, multiwell plate, chamber slides, petri dish, tube, tray, culture bag, Erlenmeyer flask, spinner flask, and roller bottle. These culture vessels are preferably non-cell-adhesive in order to enable suspension culture. As non-cell-adhesive culture vessel, those in which the surface of the culture vessel has been artificially treated (e.g., super-hydrophilic treatment with MPC polymer, low protein adsorption treatment, etc.) to reduce adhesion with cells can be used. Rotary culture may be performed using a spinner flask, roller bottle, or the like. The culture surface of the culture vessel may have a flat bottom or may have concaves and convexes.

The culture vessel used for adhesion culture is not particularly limited as long as it is capable of adhesion culture, and those skilled in the art can appropriately select a culture vessel according to the culture scale, culture conditions, and culture period. Examples of such culture vessel include flask, tissue culture flask, culture dish, tissue culture dish, multidish, microplate, microwell plate, multiplate, multi-well plate, chamber slide, petri dish, tube, tray, culture bag, micro carrier, bead, stack plate, spinner flask, and roller bottle. These culture vessels are preferably cell-adhesive in order to enable adhesion culture. As the cell adhesive culture vessels, those in which the surface of the culture vessel has been artificially treated to improve adhesion with cells can be mentioned. Specifically, surface-treated culture vessels and culture vessels whose inside is coated with a coating agent can be mentioned. Examples of culture vessels with surface treatments include culture vessels with surface treatments such as positive charge treatment. Examples of surface-treated culture vessels include culture containers subjected to surface processing such as positive electric charge treatment and the like. Examples of the coating agent include laminin [including laminin α5β1γ1 (hereinafter laminin 511), laminin α1β1γ1 (hereinafter laminin 111), etc., and laminin fragments (laminin 511E8, etc.)], entactin, collagen, fibronectin, gelatin, vitronectin, Synthemax (Corning Incorporated), extracellular matrix such as Matrigel and the like, polymers such as polylysine, polyornithine, and the like, and the like.

The neural crest cells that can be used in step 1 may be, as described in 1. Medium composition, derived from a living organism or derived from pluripotent stem cells. When using neural crest cells derived from pluripotent stem cells, cells that have just been differentiated into neural crest cells may be used or, as mentioned below, those that have been further purified or those that have undergone purification and expansion culture may be used.

As used herein, purification refers to increasing the proportion of a specific type of cell (e.g., neural crest cells) in a cell population (e.g., a cell population containing neural crest cells).

In one embodiment, the concentration of the neural crest cells to be cultured can be set to about 1×10² to about 1×10⁷ cells/cm², preferably about 3×10² to about 5×10⁶ cells/cm², more preferably about 4×10² to about 2×10⁵ cells/cm², further preferably about 4×10² to about 1×10⁵ cells/cm², further more preferably about 3×10³ to about 1×10⁴ cells/cm².

In another embodiment, the concentration of the neural crest cells to be cultured can be set to about 1×10² to about 1×10⁷ cells/cm², preferably about 3×10² to about 5×10⁶ cells/cm², more preferably about 4×10² to about 2×10⁵ cells/cm², further preferably about 4×10² to about 1×10⁵ cells/cm², further more preferably about 1.5×10⁴ to about 3×10⁴ cells/cm².

The culture conditions such as culture temperature, CO₂ concentration, and the like can be appropriately set. The culture temperature is, for example, about 30°C to about 40°C, preferably about 37°C. The CO₂ concentration is, for example, about 1% to about 10%, preferably about 5%.

Examples of the known markers of mesenchymal stem cell include CD90, CD44, CD73, and CD105. Therefore, in the cell population of mesenchymal stem cells obtained by the above-mentioned method, the majority (e.g., not less than 60%, preferably not less than 70%, more preferably not less than 80%, further preferably not less than 90%, most preferably 100%, of the cells in the cell composition) is preferably positive to any one, preferably a combination of any two, more preferably a combination of three markers, most preferably all markers, of CD90, CD44, CD73, and CD105. More preferably, the cell composition of the present invention does not express a molecule not expressed by mesenchymal stem cells. Examples of the molecule not expressed by mesenchymal stem cell include CD34 (expressed by hematopoietic stem cell), CD45 (expressed by hematopoietic stem cell), CD14 (expressed by monocyte, macrophage), CD11b (expressed by monocyte, macrophage, NK cell, granulocyte), CD79 (expressed by B cell), CD19 (expressed by B cell) and HLA-DR (expressed by dendritic cell, B cell, monocyte, macrophage) and the like. In a preferred embodiment, therefore, the majority of the cell composition of the present invention (e.g., not less than 60%, preferably not less than 70%, more preferably not less than 80%, further preferably not less than 90%, further more preferably not less than 95%, most preferably 100%, of the cells in the cell composition) is preferably negative for the expression of molecules that are not expressed in mesenchymal stem cells.

In one embodiment, the following steps may be performed before step 1:
(step A) a step of obtaining a cell population including neural crest cells, and
(step B) a step of expansion culture of the cell population obtained in step A, using an extracellular matrix as a scaffold.

In step A, as described in 1. Medium composition, a cell population containing neural crest cells can be obtained by a method known per se. In general, the proportion of neural crest cells in a cell population containing neural crest cells can vary greatly depending on the collected tissue and differentiation induction conditions. In one embodiment of the present invention, for example, the proportion of neural crest cells in a cell population containing neural crest cells can be, but is not limited to, 1 to 95%, 1 to 90%, 1 to 85%, 1 to 80%, 1 to 75%, 1 to 70%, 1 to 65%, 1 to 60%, 1 to 55%, 1 to 50%, 1 to 45%, 1 to 40%, 1 to 35%, 1 to 30%, 1 to 25%, 1 to 20%, 1 to 15%, or 1 to 10%. In another embodiment, for example, the proportion of neural crest cells in a cell population containing neural crest cells can be, but is not limited to, 25 to 95%, 25 to 90%, 25 to 85%, 25 to 80%, 25 to 75%, 25 to 70%, 25 to 65%, 25 to 60%, 25 to 55%, 25 to 50%, 25 to 45%, 25 to 40%, or 25 to 35%. In another embodiment, for example, the proportion of neural crest cells in a cell population containing neural crest cells can be, but is not limited to, 50 to 95%, 50 to 90%, 50 to 85%, 50 to 80%, 50 to 75%, 50 to 70%, 50 to 65%, or 50 to 60%. In another embodiment, for example, the proportion of neural crest cells in a cell population containing neural crest cells can be, but is not limited to, not less than 70%, 75 to 95%, 75 to 90%, or 75 to 85%.

The cell population containing neural crest cells obtained in step A may then be subjected to a step of purifying neural crest cells, and (the purified neural crest cells) may be further subjected to a step of expansion culture. That is, step B can also be called a step of purification and expansion culture of neural crest cells. In the present specification, the "expansion culture" is a concept including culture for maintaining and/or proliferating desired cells, and may preferably be culture for proliferating desired cells.

Methods for purifying neural crest cells are not particularly limited, and include, but are not limited to, for example, single cell formation and sorting processing such as sorting with a cell sorter using a fluorescence-labeled neural crest cell-specific antibody, sorting using magnetic beads bound with the aforementioned antibody, and sorting using an affinity column to which the antibody is immobilized.

In the method of purification by single cell formation, cells are subjected to dynamic dispersion treatment, dispersion treatment using enzymes such as collagenase or trypsin, and/or dispersion treatment using a chelating agent such as EDTA, as necessary, to obtain single cells. At that time, a ROCK inhibitor may be added for the purpose of suppressing cell death. The ROCK inhibitor is not particularly limited as long as it can suppress the function of Rho-kinase (ROCK), and examples thereof include Y-27632, Fasudil/HA1077, H-1152, Wf-536, and derivatives thereof. Furthermore, other known low-molecular-weight compounds can also be used as ROCK inhibitors (e.g., US-A-2005/0209261, US-A-2005/0192304, US-A-2004/0014755, US-A-2004/0002508, US-A-2004/0002507, US-A-2003/0125344, US-A-2003/0087919, and WO 2003/062227, WO 2003/059913, WO 2003/062225, WO 2002/076976, WO 2004/039796).

In the aforementioned method of purifying by sorting, for example, a CD271-specific antibody can be used as the aforementioned neural crest cell-specific antibody.

For expansion culture in step B, culture conditions suitable for maintaining and/or proliferating neural crest cells can be used.

The culture condition used for expansion culture of neural crest cells is not particularly limited as long as neural crest cells can be expansion cultured, and culture conditions known per se can be used. One example is a method for culturing cells in a culture medium containing a TGFβ inhibitor, EGF (epidermal growth factor), and FGF2 (fibroblast growth factor 2).

The medium used for expansion culture of neural crest cells can be prepared using a medium used for animal cell culture as a basal medium. Examples of the basal medium include IMDM medium, Medium199 medium, Eagle's Minimum Essential medium (EMEM) medium, αMEM medium, Dulbecco's modified Eagle's medium (DMEM) medium, Ham's F12 medium, RPMI 1640 medium, Fischer's medium, StemPro34 (Invitrogen), RPMI-base medium, StemFit (registered trademark) AK03N medium, and mixed media of these, and the like. In this step, StemFit (registered trademark) medium is preferably used. The medium may contain a serum, or may be serum-free. Where necessary, the medium may contain one or more serum replacements such as albumin, transferrin, Knockout Serum Replacement (KSR) (serum replacement for FBS during ES cell culture), N2 supplement (Invitrogen), B27 supplement (Invitrogen), fatty acid, insulin, collagen precursor, trace element, 2-mercaptoethanol (2ME), thiolglycerol, and the like, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts, and the like.

In the present invention, the TGFβ inhibitor is a substance that inhibits signal transduction from binding of TGFβ to receptors to SMAD, and is not particularly limited as long as it is a substance that inhibits binding to the ALK family (receptor) or inhibits phosphorylation of SMAD caused by the ALK family. In the present invention, the TGFβ inhibitor is exemplified by Lefty-1 (for example, as NCBI Accession No., mouse: NM_010094, human: NM_020997), SB431542, SB202190 (all above: R.K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01 (WO 2009/146408), derivatives of these, and the like. The TGFβ inhibitor used for expansion culture of neural crest cells may preferably be SB431542.

The concentration of TGFβ inhibitors such as SB431542 in a culture medium is not particularly limited as long as the concentration inhibits ALK5. It is preferably 1 nM to 50 µM, for example, 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, 50 µM, but is not limited to these. The concentration is more preferably 10 µM.

The concentration of EGF in the medium is preferably 1 ng/ml to 100 ng/ml, for example, 1 ng/ml, 5 ng/ml, 10 ng/ml, 20 ng/ml, 30 ng/ml, 40 ng/ml, 50 ng/ml, 60 ng/ml, 70 ng/ml, 80 ng/ml, 90 ng/ml, 100 ng/ml, but is not limited to these. The concentration is more preferably 20 ng/ml.

In addition, The concentration of FGF2 in the medium is preferably 1 ng/ml to 100 ng/ml, for example, 1 ng/ml, 5 ng/ml, 10 ng/ml, 20 ng/ml, 30 ng/ml, 40 ng/ml, 50 ng/ml, 60 ng/ml, 70 ng/ml, 80 ng/ml, 90 ng/ml, 100 ng/ml, but is not limited to these. The concentration is more preferably 20 ng/ml.

In addition, components other than those mentioned above can also be added to the medium as long as expansion culture of neural crest cells can be achieved.

The expansion culture of neural crest cells in step B may be performed by either adhesion culture or suspension culture, but is preferably performed by adhesion culture.

Step B is characterized by using an extracellular matrix as a scaffold to achieve purification of neural crest cells.

In the present specification, the "scaffold" refers to (i) a substance present on the surface of the cell-adhesive vessel, its materials, a surface of the cell-adhesive vessel and forming an adhesion site, and/or (ii) a substance that is dissolved, dispersed, or suspended in a cell culture medium to form a three-dimensional network in the cell culture medium, to which network the cells can adhere.

As the cell-adhesive culture vessel, one in which the surface of the culture vessel has been artificially treated to improve adhesion with cells can be mentioned. Specifically, surface-treated culture vessels and culture vessels whose inside is coated with a coating agent can be mentioned. Examples of the surface-treated culture vessel include culture vessels with surface treatments such as positive charge treatment. Examples of surface-treated incubators include culture containers subjected to surface processing such as positive electric charge treatment and the like.

Such scaffold also includes substances that are applied onto the surface of a culture container. The substance to be applied includes laminin [including laminin α5β1γ1 (laminin 511), laminin α2β1γ1 (laminin 211), laminin α1β1γ1 (laminin 111), etc. and laminin fragments (laminin 511E8, etc.)], entactin, collagen, gelatin, vitronectin, Synthemax (Corning Incorporated), extracellular matrix such as Matrigel and the like, polymers such as polylysine, polyornithine, and the like, and the like.

Such a substance that forms a three-dimensional network in a cell culture medium exhibits the effect of uniformly suspending cells and/or tissues in a liquid culture medium. More specific examples of the nanofibers contained in the medium composition of the present invention include nanofibers formed in a liquid medium by gathering and self-assembling of low-molecular-weight compounds and high-molecular-weight compounds through covalent bonding, ionic bonding, electrostatic interactions, hydrophobic interactions, van der Waals forces, and the like, and nanofibers obtained by micronizing a relatively large fiber structure made of a high-molecular-weight compound by a high-pressure treatment or the like. While not bound by theory, in the medium composition of the present invention, the nanofibers form a three-dimensional network that supports the cells and tissues, thereby maintaining the floating state of the cells and tissues.

Examples of the extracellular matrix used as a scaffold to achieve purification of neural crest cells in step B include laminin and fibronectin. In particular, laminin may be full-length laminin, laminin having α2 chain, or laminin 211.

Laminin is a glycoprotein that is a major constituent molecule of the basement membrane. It is known that laminin is involved in various cell functions such as cell adhesion, cell proliferation, metastasis, and differentiation. Laminin is composed of a heterotrimer having one each of α, β, and γ subunit chains. Currently, there are 5 types of known α subunit chains (α1, α2, α3, α4, α5), 3 types of β subunit chains (β1, β2, β3), and 3 types of γ subunit chains (γ1, γ2, γ3), and 15 types of laminin isoforms have been confirmed at present in humans, depending on the combination of these subunit chains. Laminin 211 that can be used in step B can be laminin composed of subunit chains of α2 chain, β1 chain, and γ1 chain. The origin of laminin is preferably the same as the organism from which neural crest cells are derived (e.g., when using human-derived neural crest cells, it is preferable to use human-derived laminin 211). Furthermore, it is known that laminin E8 fragment, which consists only of integrin binding sites, has stronger cell adhesion activity than full-length laminin (see Miyazaki T. et al., Nat Commun. 2012; 3:1236). The laminin 211 that can be used in step B may be a full length laminin 211 protein rather than a fragment. Laminin 211 may be prepared using gene recombination techniques known per se, or commercially available products may also be used.

In step B, when expanding the cell population in suspension culture, for example, laminin 211 may be contained in the medium so that floating cells may use same as a scaffold to form aggregates, thereby enabling three-dimensional suspension culture. Suspension culture can be performed by a method known per se. One example is a method in which a cell population is expanded in suspension culture in a medium to which laminin 211 is added, while stirring the medium using a spinner flask or the like. Alternatively, a cell population can also be expanded in suspension culture by using laminin 211 in combination with polysaccharide (e.g., methylcellulose, xanthan gum, gellan gum, etc.) that has the effect of suspending cells when added to the medium. In this embodiment, cells are dispersed in a three-dimensional spread and proliferated in the state of being attached to nanofibers or in the form of spheres. Cells attach to nanofibers and proliferate strongly while using them as scaffolds. As a result, the proliferated cells and cell aggregates (spheres, etc.) form a cluster of grapes on the nanofibers, thus enabling suspension culture of cells. The concentration of laminin to be added may be appropriately set in consideration of various conditions such as the seeding density of the cell population and the concentration of the polysaccharide to be used in combination. In the present specification, the suspension culture means a culture method in which cells are not attached to the surface of a culture container. Suspension culture may or may not involve physical stirring. Furthermore, the cells to be cultured may be uniformly dispersed in the medium or may be non-uniformly dispersed.

In step B, when expanding the cell population in adhesion culture, for example, laminin 211 is applied onto the surface of the culture container. The coating amount of laminin 211 is not particularly limited as long as the desired effect of the present invention can be obtained, and a generally recommended coating amount may be used. In one embodiment, the coating amount of laminin 211 is, but not limited to, 0.1 ng/cm² to 1000 ng/cm², preferably 0.5 ng/cm² to 500 ng/cm², more preferably 1 ng/cm² to 250 ng/cm², further preferably 2 ng/cm² to 100 ng/cm².

In addition, the culture period in step B may vary depending on the culture conditions, culture method, proportion of neural crest cells contained in the cell population, and the like, but purification of neural crest cells is achieved in a relatively short period of time. One example of the culture period is 1 to 21 days, 1 to 20 days, 1 to 19 days, 1 to 18 days, 1 to 17 days, 1 to 16 days, 1 to 15 days, 1 to 14 days, 1 to 13 days, 1 to 12 days, 1 to 11 days, 1 to 10 days, 1 to 9 days, 1 to 8 days, 1 to 7 days, 1 to 6 days, 1 to 5 days, 1 to 4 days, or 1 to 3 days, though not limited to these.

The culture temperature in step B is not particularly limited as long as neural crest cells can be cultured, and is 30 to 40°C, preferably about 37°C. The CO₂ concentration during culture in step B is not particularly limited as long as neural crest cells can be cultured, and is 2 to 5%, preferably about 5%.

In one embodiment, the following steps are performed before step 1:
(step A) a step of obtaining a cell population including neural crest cells, and
(step C) a step of expansion culture of the cell population obtained in step A, using fibronectin as a scaffold.

In step A, neural crest cells can be obtained as described in 1. Medium composition. Neural crest cells can occur in the cell population used for production. The proportion of neural crest cells in a cell population containing neural crest cells can vary greatly depending on the collected tissue and differentiation induction conditions. In one embodiment of the present invention, for example, the proportion of neural crest cells in a cell population containing neural crest cells can be, but is not limited to, 1 to 95%, 1 to 90%, 1 to 85%, 1 to 80%, 1 to 75%, 1 to 70%, 1 to 65%, 1 to 60%, 1 to 55%, 1 to 50%, 1 to 45%, 1 to 40%, 1 to 35%, 1 to 30%, 1 to 25%, 1 to 20%, 1 to 15%, or 1 to 10%. In another embodiment, for example, the proportion of neural crest cells in a cell population containing neural crest cells can be, but is not limited to, 25 to 95%, 25 to 90%, 25 to 85%, 25 to 80%, 25 to 75%, 25 to 70%, 25 to 65%, 25 to 60%, 25 to 55%, 25 to 50%, 25 to 45%, 25 to 40%, or 25 to 35%. In another embodiment, for example, the proportion of neural crest cells in a cell population containing neural crest cells can be, but is not limited to, 50 to 95%, 50 to 90%, 50 to 85%, 50 to 80%, 50 to 75%, 50 to 70%, 50 to 65%, or 50 to 60%. In another embodiment, for example, the proportion of neural crest cells in a cell population containing neural crest cells can be, but is not limited to, not less than 60%, not less than 70%, not less than 80%, for example, 75 to 95%, 75 to 90%, or 75 to 85%.

In one embodiment, cells are passaged as appropriate during step 1. Passage is performed, for example, every 2 to 8 days or every 3 to 7 days after seeding. The passage interval is preferably a period sufficient for the expansion of cell aggregates and shorter than the period in which cell aggregates become too large, making it difficult for oxygen and nutrients to reach the cells inside the cell aggregates.

The number of passages during step 1 is, for example, 0 to 20 times (0 time, 1 time, 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times), preferably 1 to 10 times, more preferably 2 to 8 times. A portion of the cell population after an appropriate number of passages may be stored as a frozen stock.

In one embodiment, cells are passaged as appropriate during step B or C. Passage is performed, for example, every 2 to 8 days or every 3 to 7 days after seeding. The passage interval is preferably a period sufficient for the expansion of cell aggregates and shorter than the period in which cell aggregates become too large, making it difficult for oxygen and nutrients to reach the cells inside the cell aggregates.

The number of passages during step B or C is, for example, 2 to 8 times (2 times, 3 times, 4 times, 5 times, 6 times, 7 times, 8 times), preferably 3 to 7 times, more preferably 4 to 6 times. A portion of the cell population after an appropriate number of passages may be stored as a frozen stock.

The transition from step B or C to step 1 is performed by replacing the culture medium with the medium composition of the present invention. The time for replacement may be after an appropriate number of passages, or during the last passage in step C. For example, it may be 6 hours before the end of the last passage in step B or C.

In one embodiment, the present invention provides a method for producing mesenchymal stem cells from a cell population containing neural crest cells, wherein
the aforementioned neural crest cells are cells induced to differentiate from pluripotent stem cells,
the aforementioned cell population is a cell population containing not less than 70% of the aforementioned neural crest cells, and the method includes a step of culturing the aforementioned cell population in the presence of corticosteroid at a converted concentration of not less than 1.25 µM and not more than 300 µM (1.25 µM to 300 µM, or not less than 1.25 µM and less than 300 µM, more than 1.25 µM and not more than 300 µM, or more than 1.25 µM and less than 300 µM).

### 3. Mesenchymal stem cell

As described above, mesenchymal stem cells are produced by culturing neural crest cells in a medium composition containing corticosteroid. The present invention also provides the thus-produced mesenchymal stem cells and cultures thereof (hereinafter also to be referred to as the mesenchymal stem cell of the present invention and a culture product thereof). The "culture product" refers to the product obtained by culturing a cell/cell population by the production method of the present invention, and includes cells, culture medium, and possibly cell-secretory components and the like.

The mesenchymal stem cell in the present invention is characterized by high expression of TSG-6 (Tumor necrosis factor (TNF)-α stimulated gene-6). TSG-6 is a member of the hyaluronan-binding protein family and contains a LINK domain that binds to hyaluronan. The expression of TSG-6 is regulated by TNF-α, IL-1, etc. TSG-6 plays roles of regulating substrate constitution and structure, controlling the interaction of chemokines with cell surface, and participating in strong anti-inflammatory action and tissue protective function, and is a property marker of mesenchymal stem cells which shows treatment effects in a wide range of disease models (Day A.J. and Milner C.M., Matrix Biol. 2019 May; 60-83).

In one embodiment, the high expression of TSG-6 is a high expression in response to IFNγ stimulation. The high expression can be detected/quantified by stimulating cells by adding IFNγ to the medium in which the mesenchymal stem cells of the present invention are cultured, and measuring the expression level of TSG-6 expressed in response to the stimulation.

In the present invention, high expression of TSG-6 means that the expression level of TSG-6 by the mesenchymal stem cells of the present invention (/expression level of TSG-6 by mesenchymal stem cells of the present invention in response to IFNγ stimulation) is higher than the expression level of TSG-6 by other mesenchymal stem cells, specifically, mesenchymal stem cells derived from living organisms, particularly mesenchymal stem cells derived from bone marrow. More specifically, high expression of TSG-6 in mesenchymal stem cells of the present invention means that the expression level is 2 times or more, 2.1 times or more, 2.2 times or more, 2.3 times or more, 2.4 times or more, 2.5 times or more, 2.6 times or more, 2.7 times or more, 2.8 times or more, 2.9 times or more, or 3 times or more, higher than the expression level of TSG-6 by other mesenchymal stem cells cultured under similar (preferably, identical) culture conditions (/stimulation with IFNγ) in the medium composition of the present invention.

The aforementioned TSG-6 protein can be isolated and purified from mesenchymal stem cells of the present invention by methods well known in the art.

Secretome can also be isolated and purified from mesenchymal stem cells of the present invention by methods well known in the art.

As used in the present specification, secretome is a concept including exosomes and all extracellular vesicles with various sizes and production processes which are other than exosomes that may be included during preparation, and also protein components contained in cell culture supernatants. Exosomes are about 100 nm in size and contain cholesterol, sphingomyelin, ceramide, and lipid raft components in the membrane, as well as many proteins, mRNA, miRNA, and the like inside. Secretome secreted from the mesenchymal stem cells of the present invention has an antifibroticaction action.

### 4. Medicament for prophylaxis or treatment of diseases

Furthermore, the present invention also provides a medicament for the prophylaxis or treatment of inflammatory diseases or fibrous diseases, containing, as an active ingredient, the mesenchymal stem cell of the present invention, TSG-6 protein isolated/purified from the mesenchymal stem cell of the present invention, or secretome isolated/purified from the mesenchymal stem cell of the present invention.

In one embodiment, the medicament for the prophylaxis or treatment of present invention is obtained, without limitation, by suspending the mesenchymal stem cells obtained above in physiological saline or an appropriate buffer (e.g., phosphate buffered saline). In this case, when the number of the obtained mesenchymal stem cells is low, they may be cultured and allowed to proliferate until a predetermined number of cells is obtained. The culture of the obtained mesenchymal stem cells is not particularly limited, but can be performed in general proliferation medium, for example, DMEM, EMEM, RPMI-1640, F-12, α-MEM, MSC growing medium (Bio Whittaker). The culture temperature is generally about 30 to 40°C, preferably about 37°C. The CO₂ concentration is generally about 1 to 10%, preferably about 5%. The humidity is generally about 70 to 100%, preferably about 95 to 100%.

When mesenchymal stem cells are used in a medicament for the prophylaxis or treatment of diseases, dimethyl sulfoxide (DMSO), serum albumin, and the like may be included in the cell preparation to protect the cells and antibiotics and the like may be included to prevent bacterial contamination and growth. Furthermore, pharmaceutically acceptable other components (e.g., carrier, excipient, disintegrant, buffering agent, emulsifier, suspension, soothing agent, stabilizer, preservative, antiseptic, physiological saline, and the like) may be included in the cell preparation. The skilled artisan can add these factors and agents to the cell preparation in appropriate concentrations.

The number of mesenchymal stem cells contained in the prophylactic or therapeutic medicament prepared above can be appropriately adjusted in consideration of the gender, age, body weight, and condition of affected area of the subject, condition of cells used etc. so that the desired effect can be obtained in the treatment of inflammatory diseases or fibrotic diseases. The target individuals include, but are not limited to, mammals such as humans. The cell preparation of the present invention may be administered multiple times (e.g., 2 to 10 times) at appropriate intervals (e.g., twice a day, once a day, twice a week, once a week, once every two weeks, once a month, once every two months, once every three months, once every 6 months) until the desired therapeutic effect is obtained. Therefore, depending on the condition of the subject, a therapeutically effective amount includes, for example, 1 to 10 doses of 1×10³ cells to 1×10¹⁰ cells per individual per administration. The total amount administered to one individual includes, but is not limited to 1×10³ cells to 1×10¹¹ cells, preferably 1×10⁴ cells to 1×10¹⁰ cells, further preferably 1×10⁵ cells to 1×10⁹ cells, and the like.

In another embodiment, the medicament for the prophylaxis or treatment of inflammatory diseases of the present invention contains a TSG-6 protein isolated/purified from the mesenchymal stem cell of the present invention as an active ingredient. The content of the TSG-6 protein in the prophylactic or therapeutic medicament varies depending on the dosage form, dose of TSG-6 protein, and the like. It is, for example, about 0.1 to 100 wt%. While the dose of TSG-6 protein is not limited, it is, for example, 0.01 ng/kg to 1 mg/kg or 10 ng/kg to 100 µg/kg.

In still another embodiment, the medicament for the prophylaxis or treatment of fibrous diseases of the present invention contains secretome isolated/purified from the mesenchymal stem cell of the present invention as an active ingredient. The content of secretome in the prophylactic or therapeutic medicament varies depending on the dosage form, dose of secretome, and the like. It is, for example, about 0.1 to 100 wt% in the protein amount. While the dose of secretome is not limited, it is, for example, 0.01 ng/kg to 1 mg/kg or 10 ng/kg to 100 µg/kg.

Examples of the inflammatory diseases to be prevented or treated by the medicament for the prophylaxis or treatment of inflammatory diseases of the present invention include hepatitis, cirrhosis, Crohn's disease, rheumatoid arthritis, Behcet's disease (ocular symptoms), ulcerative colitis, ankylosing spondylitis, psoriasis (including psoriatic arthritis), HIV infectious disease, multiple myeloma, congestive cardiac failure, GVHD, giant cell arteritis (GCA), polymyalgia rheumatica (PMR), pigmented purpuric lichenoid dermatitis, sarcoidosis, Wegener's granulomatosis, pyoderma, Behcet's disease, TNF receptor-associated periodic syndrome (TRAPS), SAPHO syndrome, Takayasu disease, myositis, Still's disease, periarteritis nodosa (PN), relapsing polychondritis, scleroderma polymyositis, hemophagocytosis syndrome, pemphigus, and Kawasaki disease atopic dermatitis.

Examples of the fibrous diseases to be prevented or treated by the medicament for the prophylaxis or treatment of fibrous diseases of the present invention include, in relation to skin, scleroderma (including localized scleroderma, generalized morphea, and linear scleroderma), keloidal scarring, psoriasis, hypertrophic scarring due to burn, atherosclerosis, restenosis, and fibrosis arising from conditions including pseudoscleroderma caused by spinal cord injury, kidney fibrosis (including glomerulosclerosis, renal tubule stroma fibrosis, progressive renal diseases and diabetic nephropathy), cardiac fibrosis (e.g., cardiac muscle fibrosis), lung fibrosis (e.g., glomerulosclerosis lung fibrosis, idiopathic lung fibrosis, silicosis, asbestosis, interstitial lung disease, interstitial fibrotic lung disease, and lung fibrosis induced by chemotherapy/radiation irradiation), mouth cavity fibrosis, endomyocardial sclerosis, deltoid fibrosis, pancreatitis, inflammatory colitis, Crohn's disease, nodular fasciitis, eosinophilic fasciitis, common fibrosis syndrome characterized by replacement of normal muscle tissue by fibrous tissue at various degrees, retroperitoneal fibrosis, cirrhosis, chronic renal failure; myelofibrosis, collagen accumulation colitis, and acute fibrosis, and in relation to eye, cornea fibrosis, cornea scarring due to surgery, trabeculectomy-induced fibrosis, and fibrosis arising from conditions including other ocular fibrosis.

The administration method of the medicament for the prophylaxis or treatment of the present invention is not particularly limited. Examples thereof include intravascular administration (preferably intravenous administration), intraperitoneal administration, intestinal administration, subcutaneous administration, topical administration to affected part, and the like preferably, intravascular administration and topical administration more preferably.

### 5. Method for prophylaxis or treatment of disease

Furthermore, the present invention provides a method for the prophylaxis or treatment of inflammatory diseases or fibrous diseases, including administering to test subjects a therapeutically effective amount of the mesenchymal stem cells of the present invention and/or the medicament for the prophylaxis or treatment of inflammatory diseases or fibrous diseases of the present invention.

In the present specification, the "effective amount" means the amount of the active ingredient that produces the desired effect.

The "therapeutically effective amount" used in the present specification means the amount of active ingredient that produces the desired therapeutic effect when administered to a test subject. The therapeutically effective amount may be administered (transplanted) at once, or may be administered (transplanted) in multiple doses. The number of times the transplantation is applied is determined by the health care provider and guidelines and according to the disease. When transplantation is performed multiple times, the interval is not particularly limited and may be several days to several weeks. The dosage is similar to that of mesenchymal stem cells and the like described in the above-mentioned 4. Medicament for prophylaxis or treatment of diseases.

Examples of the animal to be the subject of the cell medicine include experiment animals such as rodents (e.g., mouse, rat, hamster, guinea pig and the like), rabbit and the like, domestic animals such as swine, bovine, goat, horse, sheep, mink and the like, pets such as dog, cat and the like, primates such as human, monkey, Macaca mulatta, marmoset, orangutan, chimpanzee and the like, and the like, preferably human.

The range of disease sites to which the mesenchymal stem cells of the present invention can be applied is appropriately selected according to the target disease, animal species, age, gender, body weight, and condition of the subject of administration, and the like.

### 6. Production method of medicament for prophylaxis or treatment of disease

The present invention provides a method for producing a medicament for the prophylaxis or treatment of inflammatory diseases or fibrous diseases, which contains mesenchymal stem cells as an active ingredient (hereinafter the production method of the medicament for the prophylaxis or treatment of diseases of the present invention), the method including a step of culturing neural crest cells in the medium composition of the present invention to induce mesenchymal stem cells (step 1). The step 1 in the production method of the medicament for the prophylaxis or treatment of diseases of the present invention can be performed in the same manner as in step 1 of the above-mentioned production method of mesenchymal stem cells of the present invention.

In the production method of the medicament for the prophylaxis or treatment of diseases of the present invention, neural crest cells to be induced to differentiate into mesenchymal stem cells (pluripotent stem cells when the neural crest cells are derived from pluripotent stem cells; somatic cells to be induced into iPS cells when the pluripotent stem cells are iPS cells) may be either autologous or allogeneic (homologous or heterologous) to the subject of administration, and the individual as the source of the cells is not particularly limited. For example, when the medicament of the present invention is produced for administration to animals in need of prophylaxis or treatment of disease, these cells may have tissue compatibility to the extent that mesenchymal stem cells derived from donor cells can be engrafted in the recipient.

For example, when the medicament for the prophylaxis or treatment of diseases of the present invention is used for the prophylaxis or treatment of a disease in a human, from the aspect of preventing death by the attack of the immune system, these cells can be the patient' s own cells or harvested from others with HLA types that are identical or substantially identical to the patient's HLA type. The "substantially identical HLA type" used in the present specification means that the donor's HLA type matches that of a patient to the extent that mesenchymal stem cells derived from the donor's cells can be engrafted when transplanted into the patient with the use of immunosuppressant and the like. For example, HLA type in which the main HLAs (three major loci of HLA-A, HLA-B, and HLA-DR, or four loci including HLA-Cw as well) are identical and the like can be mentioned.

The production method of the medicament for the prophylaxis or treatment of diseases of the present invention may include a step of adding dimethyl sulfoxide (DMSO), serum albumin, and the like to the medicament in order to protect the mesenchymal stem cells and antibiotics and the like in order to prevent bacterial contamination and growth. Furthermore, pharmaceutically acceptable other components (e.g., carrier, excipient, disintegrant, buffering agent, emulsifier, suspension, soothing agent, stabilizer, preservative, antiseptic, physiological saline, and the like) may be included in the medicament. The skilled artisan can add these factors and agents to the medicament in appropriate concentrations.

In one embodiment, the production method of the medicament for the prophylaxis or treatment of inflammatory diseases of the present invention further includes collecting TSG-6 protein from the induced mesenchymal stem cells and/or culture supernatants, and the medicament for the prophylaxis or treatment of inflammatory diseases contains TSG-6 protein as an active ingredient.

In one embodiment, the production method of the medicament for the prophylaxis or treatment of fibrous diseases of the present invention further includes collecting secretome from the induced mesenchymal stem cells and/or culture supernatants, and the medicament for the prophylaxis or treatment of fibrosis contains the secretome as an active ingredient.

The present invention is further described in detail in the following examples, but the present invention is not limited in any way by these examples.

### [Example]

### Example 1: Differentiation induction from iNCC (iPS cell-derived neural crest cell) into iMSC (iPS cell-derived mesenchymal stem cell)

### 1. Differentiation induction from iPS cell into neural crest cell (iNCC)

201B7 (iPS portal) was used as the iPS cell line. iPS cells were seeded at 6500 cells/well in a 6-well plate coated with laminin 511-E8 fragment (iMatrix511, Nippi), and cultured at 37°C under 5% CO₂ for 5 days in StemFit (registered trademark) AK03N (Ajinomoto Co., Inc.) medium. Then differentiation into neural crest cells was induced at 37°C under 5% CO₂ for 14 days in a medium obtained by adding SB431542 (Stemgent, Inc., 10 µM) and CHIR99021 (Wako, 0.3 µM (condition 1) or 0.9 µM (condition 2)) to StemFit AK03N (solution A + solution B). The percentage of neural crest cells at the end of induction was calculated by determining using FACS the proportion of cells highly expressing CD271 protein. In addition, the gene expression state of neural crest cell markers was investigated by an RT-PCR method. The primers used in the RT-PCR method are shown below.

| marker gene | Primer ID | Taqman Cat.No. |
|---|---|---|
| TFAP2a | Hs00271528_CE | A15629 |
| SOX9 | Hs01001343_g1 | 4331182 |
| TWIST1 | Hs01675818_s1 | 4331182 |

### (β actin was used as reference gene (Hs01101944_s1, 4331182))

### 2. Purification and expansion culture of neural crest cells (iNCC)

The cell population containing the neural crest cells produced in the above-mentioned 1. was converted into single cells using TrypLE Select (Thermo Fisher). The single cell population was seeded onto a 6-well plate coated with a scaffold material, and cultured under culture conditions suitable for expanding neural crest cells. More specifically, the cells were cultured at 37°C under 5% CO₂ for 9 to 10 days in a medium obtained by adding SB431542 (10 µM), Epithelium growth factor (Sigma Chemical, 20 ng/mL), and StemFit AK03N solution C (Ajinomoto Co., Inc., 0.08%) to StemFit AK03N (solution A + solution B). After culturing for 9 to 10 days, when the confluency reached approximately 90%, the proportion of neural crest cells and expression state of neural crest cell gene markers in the cell population cultured on scaffold material were determined by the same method as described in the above-mentioned 1.

As the scaffold material, laminin 211 (Biolamina, 6.3 ng/cm² and 62.5 ng/cm²) was used.

The scaffold material was coated on the surface of 6-well plates by direct suspension in culture medium.

### 3. Differentiation induction from iNCC into iMSC

The obtained iNCCs (iPSC-derived NCCs) were seeded at a density of 5.0×10³ cells/well into a 6-well plate coated with vitronectin-N (Thermo Fisher Scientific) at a density of 1.5 µg/cm² and cultured under 37°C, 5% CO₂ conditions for 13 days using the following differentiation medium (T3 medium) to induce iMSCs (iPSC-derived MSCs).

### T3 medium: StemFit (registered trademark) For Mesenchymal Stem Cells (Ajinomoto Co., Inc.), 90 nM Dexamethasone (Sigma-Aldrich)

The induced iMSCs were subjected to surface antigen analysis using FACS using three types of MSC positive markers (CD90, CD44, CD73) and one type of negative marker (CD34). The analysis results are shown in Table 1.

**[Table 1]**

| | | FACS positive rate [%] |
|---|---|---|
| MSC marker | CD90 | 99.7 |
| | CD44 | 100 |
| | CD73 | 99.8 |
| blood cell marker | CD34 | 3.8 |

The obtained cells were positive for CD90, CD44, and CD73 and negative for CD34, confirming iMSC induction.

### Example 2: Analysis of inflammatory stimulus responsiveness of iMSCs by IFNγ stimulation

iNCCs were seeded at a density of 5.0×10³ cells/well into a 6-well plate coated with vitronectin-N at a density of 1.5 µg/cm², and cultured under 37°C, 5% CO₂ conditions for 16 days using T3 medium to induce iMSCs.

After 16 days from the start of induction, 50 ng/mL IFNγ (R&D Systems) was added to T3 medium to stimulate for 48 hr. After stimulation, RNA was purified according to the protocol of PureLink (trademark) RNA Mini Kit (Thermo Fisher Scientific) and, after reverse transcription, gene expression of TSG-6 (TNFAIP6), a therapeutic marker for inflammatory diseases, was analyzed using Real-Time PCR. As comparison targets, iNCC-derived iMSC induced to differentiate in αMEM medium containing 10% FBS, and two lots of living bone marrow-derived MSC (BMMSC) (Lonza or RoosterBio) cultured in T3 medium for 12 to 16 days were used (Table 2).

**[Table 2]**

| cell type | derived from | proliferation or induction medium |
|---|---|---|
| iMSC (T3) | iPSC | T3 medium |
| iMSC (FBS) | iPSC | αMEM containing 10% FBS |
| BMMSC_1 | human bone marrow | T3 medium |
| BMMSC_2 | human bone marrow | T3 medium |

The gene expression analysis results of TSG-6 are shown in Fig. 1. iMSC induced in T3 medium showed significantly increased gene expression of TSG-6 by IFNγ stimulation.

### Example 3: Examination of Dexamethasone concentration

iNCCs were seeded at a density of 3.0×10⁴ cells/well into a 6-well plate coated with vitronectin-N at a density of 1.5 µg/cm², and cultured under 37°C, 5% CO₂ conditions using StemFit (registered trademark) For Mesenchymal Stem Cells supplemented with 50 nM, 100 nM, 1 µM, 10 µM, 50 µM Dexamethasone to induce iMSCs. After 18 days from the start of induction, 50 ng/mL IFNγ was added to each medium to stimulate for 48 hr. After stimulation, RNA was purified and, after reverse transcription, gene expression of TNFAIP6 was analyzed using Real-Time PCR.

Fig. 2 shows the cell proliferation curve during differentiation induction. Proliferation of cells in the group added with 50 µM Dexamethasone stopped after day 13.

The gene expression analysis results of TSG-6 are shown in Fig. 3. iMSC induced by adding 50 nM to 10 µM Dexamethasone showed significantly increased gene expression of TSG-6 by IFNγ stimulation.

### Example 4: Examination of type of corticosteroid

iMSC was induced according to Example 1, except that 100 nM Dexamethasone, 100 nM betamethasone, 100 nM predonisolone, 666 nM predonisolone were used.

Regarding the induced iMSC, surface antigen analysis of 4 types of MSC positive markers (CD90, CD44, CD73, CD105), 1 type of NCC positive marker (MSC micro-positive marker) (CD271), and two types of MSC negative markers (CD45 and CD34) was performed using FACS. The analysis results are shown in Table 3.

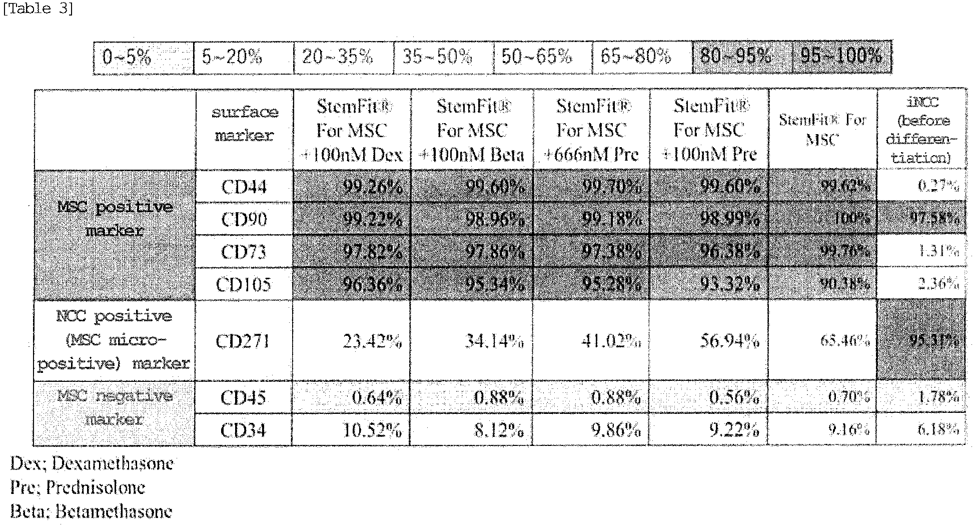

Prednisolone, like Dexamethasone, had a limited effect when added at 100 nM, but was effective in promoting differentiation when the titer as glycocorticoid was aligned at 666 nM.

The converted concentrations of Dexamethasone 50 nM, 90 nM, 100 nM, 1 µM, 10 µM, 50 µM in this Example were respectively 1.333 µM 2.3994 µM, 2.666 µM, 26.66 µM, 266.6 µM, 1333 µM (converted with Dexamethasone titer as 26.66).

The converted concentrations of prednisolone 100 nM, 666 nM in this Example were respectively 400 nM, 2.664 µM (converted with predonisolone titer as 4). The converted concentration of betamethasone 100 nM in this Example was 2.666 µM (converted with betamethasone titer as 26.66).

### Example 5: iMSC secretome evaluation

### (Materials and method)

### 1. Collection of iMSC secretome

Secretome is a protein secreted from cells and is known to include exosome and the like. The iMSC frozen stock at passage number p1 was cultured for 3 passages using StemFit (registered trademark) For Mesenchymal Stem Cell (Ajinomoto Co., Inc.) supplemented with 90 nM Dexamethasone (Sigma-Aldrich). After medium change to DMEM/F-12 (Gibco) medium at 80% confluency, the culture was grown for 2 days and the culture supernatant was collected. The culture supernatant was subjected to 0.22 um filtering. Thereafter, it was filled in a Centricon (registered trademark) Plus-70 column and ultrafiltration was performed by centrifugation at 3,500×g for 25 min. The concentrated culture supernatant was collected by turning the column upside down and centrifuging at 1,000×g for 2 min. The obtained secretome is shown in Table 4.

**[Table 4]**

| group name | concentration source liquid | concentration rate |
|---|---|---|
| medium | DMEM/F-12 | 145 |
| secretome | culture supernatant obtained by culturing iMSC in DMEM/F-12 for 2 days | 71 |

### 2. Evaluation of antifibroticaction action of secretome obtained from iMSC

LX-2 Human Hepatic Stellate Cell Line (EDM Millipore, hereinafter LX-2 cells) was cultured in a DMEM high Glucose (Life Technologies) medium supplemented with 10% FBS (Gibco), 1% penicillin-streptomycin (Nacalai), and 2 mM Glutamine (Gibco). On the fourth passage, 4×10⁴ cells were seeded into each well of a 12-well plate. After 2 days of culture, the medium was replaced with a medium in which concentrated secretome obtained from iMSC was added at a concentration of 1× to a medium supplemented with 10 ng/mL TGF-β1 (Peprotech), and the culture was continued. On the second day after replacing the medium, cells were detached with Accutase (Thermo Fisher), and after collection, RNA was extracted and purified using Maxwell (registered trademark) 16 LEV simplyRNA Purification Kits (Promega), and reverse transcription was performed using PrimeScripttm RT Master Mix (Takara). RT-PCR was performed on the synthesized cDNA using the primers of TaqMan (registered trademark) Gene Expression Assays shown in Table 5.

**[Table 5]**

| Gene | | Assay ID |
|---|---|---|
| GAPDH | | Hs02758991_g1 |
| COL1A1 | | Hs00164004_m1 |
| ACTA2 | | Hs00426835_g1 |

### (Results)

The results of RT-PCR are shown in Fig. 4. TGF-β1 stimulation markedly increased expression of fibrosis marker genes COL1A1 and ACTA2 in LX-2 cells. In contrast, the addition of DMEM/F12 itself had no particular effect, but iMSC secretome reduced COL1A1 and ACTA2 expression which increases by TGF-β1 stimulation. Furthermore, iMSC secretome also reduced COL1A1 and ACTA2 expression in TGF-β1 unstimulated LX-2 cells.

### [Industrial Applicability]

According to the present invention, a medium composition for efficiently inducing mesenchymal stem cells from neural crest cells can be provided. According to the present invention, moreover, an efficient production method of mesenchymal stem cells, a medicament for the prophylaxis or treatment of inflammatory diseases or fibrous diseases, containing the mesenchymal stem cells and the like as active ingredients, a prophylactic or therapeutic method for the diseases, a production method of a medicament for the treatment, and the like can be provided.

This application is based on a patent application No. 2021-097619 filed in Japan (filing date: June 10, 2021), the contents of which are incorporated in full herein.

## Claims

1. A method for producing a medicament for the prophylaxis or treatment of inflammatory diseases or fibrous diseases, comprising a step of inducing mesenchymal stem cells by culturing neural crest cells in a medium composition for inducing mesenchymal stem cells from neural crest cells, comprising a basal medium and corticosteroid, the composition having a converted concentration of the corticosteroid in the medium composition of not less than 1.25 µM.

2. The method according to claim 1, wherein the medicament for the prophylaxis or treatment of inflammatory diseases or fibrous diseases comprises a mesenchymal stem cell **characterized by** high expression of TSG-6 as an active ingredient.

3. The method according to claim 1, which is a method for producing a medicament for the prophylaxis or treatment of inflammatory diseases, further comprising collecting TSG-6 protein from the induced mesenchymal stem cells and/or culture supernatant, wherein the medicament for the prophylaxis or treatment of inflammatory diseases comprises TSG-6 protein as an active ingredient.

4. The method according to claim 1, which is a method for producing a medicament for the prophylaxis or treatment of fibrous diseases, further comprising collecting secretome from the induced mesenchymal stem cells and/or culture supernatant, wherein the medicament for the prophylaxis or treatment of fibrosis comprises the secretome as an active ingredient.

5. The production method according to any one of claims 1 to 4, wherein the corticosteroid is glucocorticoid or a derivative thereof.

6. The production method according to claim 5, wherein the glucocorticoid or a derivative thereof is at least one member selected from the group consisting of cortisone acetate, hydrocortisone, fludrocortisone acetate, predonisolone, triamcinolone, methylprednisolone, dexamethasone, betamethasone, and beclometasone dipropionate.

7. The production method according to claim 5 or 6, wherein the glucocorticoid or a derivative thereof is dexamethasone.

8. The production method according to any one of claims 1 to 7, wherein the converted concentration of the corticosteroid in the medium composition is not more than 300 µM.

9. The production method according to claim 7, wherein the concentration of dexamethasone in the medium composition is not more than 10 µM.

10. The production method according to any one of claims 1 to 9, wherein the basal medium is a serum-free medium.

11. The production method according to any one of claims 1 to 10, wherein the neural crest cell is derived from a pluripotent stem cell.

12. The production method according to claim 11, wherein the pluripotent stem cell is an induced pluripotent stem cell (iPS cell).

13. The production method according to any one of claims 1 to 12, wherein the following steps are performed before the
aforementioned step:
(step A) a step of obtaining a cell population including neural crest cells, and
(step B) a step of expansion culture of the cell population obtained in step A, using an extracellular matrix as a scaffold.

14. The production method according to claim 13, wherein the extracellular matrix is laminin or fibronectin.

15. The production method according to claim 14, wherein the laminin is a full-length laminin, laminin having an α2 chain or laminin 211.

16. The production method according to any one of claims 13 to 15, wherein the cell population including neural crest cells obtained in step A is subjected to step B without being subjected to a neural crest cell sorting treatment.

17. The production method according to any one of claims 1 to 3 and 5 to 16, wherein the inflammatory disease is cirrhosis.

18. The production method according to any one of claims 2, 3, and 5 to 17, wherein the high expression of TSG-6 is a high expression in response to IFNy stimulation.

19. A medicament for the prophylaxis or treatment of inflammatory diseases produced by the production method according to any one of claims 1 to 3 and 5 to 18, or a medicament for the prophylaxis or treatment of fibrous diseases produced by the production method according to any one of claims 1, 2, and 4 to 18.
